# EUROPÄISCHE PATENTANMELDUNG

(11) **EP 1 629 826 A1**
(43) Veröffentlichungstag der Anmeldung: **01.03.2006**
(21) Anmeldenummer: 05017723.7
(22) Anmeldetag: 16.08.2005
(51) Int. Cl.: A61K 8/66, A61K 8/97, A61Q 5/10

(54) **Oxidatives Färbeverfahren für keratinische Fasern**

(30) Priorität: 26.08.2004 DE 102004041567
(71) Anmelder: Henkel Kommanditgesellschaft auf Aktien, 40589 Düsseldorf (DE)
(72) Erfinder: Sättler, Andrea, 40225 Düsseldorf (DE); Kleen, Astrid, 22763 Hamburg (DE); Höffkes, Horst, 40595 Düsseldorf (DE); Otto, Ralf, 88422 Oggelshausen (DE); Gerke, Thomas, 41468 Neuss (DE)

(57) **Zusammenfassung**

Die vorliegende Erfindung betrifft Mittel zur Färbung keratinischer Fasern, enthaltend in einem physiologisch akzeptablen Medium mindestens ein Farbstoffvorprodukt sowie mindestens einen Pflanzensaft sowie Mittel zur Färbung keratinischer Fasern, enthaltend in einem physiologisch akzeptablen Medium mindestens ein Farbstoffvorprodukt sowie zumindest die Enzymfraktion sowie die natürlichen Aromastoffe eines Pflanzensaftes. Die erfindungsgemäßen Mittel erfüllen die Anforderungen an Oxidationsmittel für eine oxidative Farbentwicklung in einem hohen Maße.

## Beschreibung

Die vorliegende Erfindung betrifft Mittel zum Färben keratinischer Fasern, die mindestens ein Farbstoffvorprodukt sowie als Oxidationsmittelzubereitung einen Pflanzensaft enthalten, ein Verfahren zur Färbung keratinischer Fasern unter Verwendung dieses Mittels sowie die Verwendung von Pflanzensäften zur oxidativen Färbung keratinischer Fasern.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Die oxidative Entwicklung der Färbung kann grundsätzlich mit Luftsauerstoff erfolgen. Bevorzugt wird jedoch ein chemisches Oxidationsmittel eingesetzt. Als Oxidationsmittel kommen Persulfate, Chlorite und insbesondere Wasserstoffperoxid oder dessen Anlagerungsprodukte an Harnstoff, Melamin sowie Natriumborat in Frage. Üblicherweise wird eine etwa 2-9%ige wässrige Wasserstoffperoxidlösung eingesetzt. Unter der Einwirkung derart hoher Oxidationsmittelkonzentrationen können die keratinischen Fasern, insbesondere wenn sie bereits dauergewellt oder gebleicht sind, geschädigt werden, und in seltenen Fällen können durch diese hohen Konzentrationen auch Hautirritationen auftreten.

Ein wesentlicher Lösungsansatz zu dieser Problematik geht von der Reduzierung der Oxidationsmittelkonzentration aus. Es wurde daher in der Vergangenheit einerseits nach Farbstoffvorprodukten gesucht, die aufgrund ihrer chemischen Struktur bereits durch geringere Mengen Wasserstoffperoxid oder durch Luftsauerstoff oxidiert werden können. Andererseits wurde die Verwendung von Enzymen als Biokatalysatoren vorgeschlagen, die den erwünschten Oxidationsprozess mit sehr wenig oder ganz ohne Wasserstoffperoxid nur in der Anwesenheit von Luftsauerstoff katalysieren können.

So wird beispielsweise in der deutschen Offenlegungsschrift DE-OS-2 155 390 ein enzymaktiviertes, oxidatives Haarfärbeverfahren beschrieben, bei dem geringe Mengen H₂O₂ in Kombination mit einem Peroxidase-Enzym eingesetzt werden. Auch in der EP-A1-0 310 675 werden enzymatische Haarbehandlungsmittel offenbart, die mindestens eine Dielektronen-reduzierende Oxidase, die Sauerstoff als Akzeptor nutzt, enthalten. In der EP-B1-0 548 620 werden enzymatische Haarfärbemittel beschrieben, bei denen die Oxidation der Farbstoffvorprodukte durch Einsatz einer Peroxidase katalysiert wird. Schließlich werden in der EP-A2-0 795 313 enzymatische Haarfärbemittel beschrieben, die ein Sauerstoff-Oxidoreduktase/Substrat-System und eine Peroxidase sowie als Kupplerkomponenten zwingend ein m-Phenylendiaminderivat enthalten. In jüngster Zeit sind ferner eine Vielzahl von Patentanmeldungen erschienen, die sich mit der Formulierung spezieller Anwendungszubereitungen auf Basis der Enzyme beschäftigen. Alle diese Färbemittel können aber bezüglich der erzielbaren Färbeleistung (Intensität, Nuance, Glanz, Echtheitseigenschaften) noch nicht vollständig überzeugen. Ferner sind beim Einsatz von Enzymen in kosmetischen Mitteln auch immer häufiger toxikologische Fragestellungen von Relevanz. Ein weiterer Nachteil enzymatischer Färbesysteme ist der erhöhte Preis, der nicht zuletzt durch den Einsatz der aufgereinigten Enzyme verursacht wird.

Die Technik der leicht-oxidierbaren Farbstoffvorprodukte hat ebenso wie die bislang beschriebene enzymatische Farbentwicklung den Nachteil, dass im Vergleich zu den herkömmlichen Verfahren insbesondere bezüglich der Intensität, des Glanzes und der Echtheitseigenschaften der Färbungen schlechtere Ergebnisse erzielt werden.

Im Rahmen der umfangreichen Untersuchungen, die der nunmehr vorliegenden Anmeldung zugrunde liegen, wurde überraschenderweise gefunden, dass Pflanzensäfte die Anforderungen an Oxidationsmittel für eine oxidative Farbentwicklung in einem hohen Maße erfüllen.

Ein erster Gegenstand der vorliegenden Erfindung sind daher Mittel zur Färbung keratinischer Fasern, enthaltend in einem physiologisch akzeptablen Medium mindestens ein Farbstoffvorprodukt sowie mindestens einen Pflanzensaft.

Ein zweiter Gegenstand der vorliegenden Erfindung sind Mittel zur Färbung keratinischer Fasern, die zumindest die Enzymfraktion sowie die natürlichen Aromastoffe eines Pflanzensaftes enthalten.

Dass aus Pflanzensäften Enzyme gewonnen werden können, die in aufgereinigter Form Farbstoffvorprodukte oxidieren können, ist prinzipiell literaturbekannt. Da Pflanzensäfte jedoch eine komplexe Mischung diverser Komponenten darstellen und beispielsweise Antioxidantien enthalten, die die Pflanzensäfte stabilisieren, war es für den Fachmann überraschend, dass auch die vollständigen, das heißt nicht aufgereinigten, Pflanzensäfte für diese Zwecke eingesetzt werden können.

Unter keratinischen Fasern sind erfindungsgemäß Pelze, Wolle, Federn und insbesondere menschliche Haare zu verstehen.

Unter einem Pflanzensaft ist erfindungsgemäß der Saft zu verstehen, der erhalten wird, wenn die Früchte, Blätter und/oder Stämme von Pflanzen gegebenenfalls mechanisch zerkleinert und anschließend die festen Bestandteile der Frucht, Blätter und/oder Stämme mit rein mechanischen Trennmethoden abgetrennt werden. Eine Gewinnung des Saftes aus den Früchten ist erfindungsgemäß besonders bevorzugt. Im Rahmen der Gewinnung des Saftes ist es dabei erfindungsgemäß zulässig, zu jedem beliebigen Zeitpunkt gegebenenfalls den pH-Wert der Zubereitung zu regulieren und/oder gegebenenfalls Stabilisatoren hinzuzugeben.

Als Beispiele für mechanische Zerkleinerungsmethoden sei beispielsweise Zerschneiden, Pürieren, Zerpressen, Mörsern und Zermahlen genannt. Zerschneiden, Pürieren und Zerpressen sind erfindungsgemäß besonders bevorzugt. Es kann erfindungsgemäß bevorzugt sein, das Pflanzengewebe zu pressen, den Presssaft zu verwerfen und das Gewebe einer zweiten Pressung zu unterziehen. Dieses Procedere kann erfindungsgemäß mehrfach wiederholt werden; dabei hat sich herausgestellt, dass die weiteren Presssäfte kleinvolumiger aber höher konzentriert sind. Es kann daher erfindungsgemäß bevorzugt sein eine spätere Fraktion eines solchem Mehrfachpressung einzusetzen.

Unter mechanischen Trennmethoden sind erfindungsgemäß solche Trennmethoden zu verstehen, die auf der Ausnutzung von mechanischen Kräften, wie beispielsweise Schwerkraft, Zentrifugalkraft, Druck oder Vakuum, beruhen. Zu diesen gehören beispielsweise Dekantieren, Filtration, Sedimentation, Ultrafiltration und Zentrifugieren. Explizit erfindungsgemäß in dieser Ausführungsform nicht umfasst sind Zubereitungen die aus Pflanzenfrüchten durch chemische Trennmethoden, wie beispielweise chromatographische Verfahren, erhalten werden.

Die pH-Wert-Regulierung spielt eine wichtige Rolle zur Stabilisierung der Enzyme und kann erfindungsgemäß mittels Zugabe von kosmetisch verträglichen Basen oder Säuren erfolgen. Ferner kann es erfindungsgemäß weiterhin bevorzugt sein, den Pflanzensaft mittels eines Säure/Base-Puffers auf einen bestimmten pH-Bereich einzustellen. Ein pH-Bereich von 5 - 7 ist erfindungsgemäß bevorzugt, ein Bereich von 7 bis 7,8 ist dabei besonders bevorzugt.

Bevorzugte Säuren sind beispielsweise die organischen Säuren Citronensäure, Äpfelsäure, Maleinsäure, Malonsäure, Itaconsäure, Weinsäure, Oxalsäure, Glutarsäure, Glutaminsäure, Milchsäure, Fumarsäure, Glykolsäure, Essigsäure sowie deren Mischungen. Eine besonders bevorzugte Organische Säure ist Citronensäure. Weiterhin können aber auch anorganische Säuren zur Einstellung des pH-Wertes verwendet werden, wie beispielsweise Salzsäure, Phosphorsäure oder Schwefelsäure. Eine besonders bevorzugte anorganische Säure ist die Salzsäure.

Als Basen sind erfindungsgemäß Alkalimetallsilikate, Alkali- und Erdalkalimetallhydroxide, Carbonate, Hydrogencarbonate sowie deren Mischungen bevorzugt. Carbonate, Hydrogencarbonate und Alkalihydroxide, wie beispielsweise Natriumhydroxid, sind erfindungsgemäß besonders bevorzugt. Weitere erfindungsgemäß bevorzugt Basen sind Ammoniak sowie primäre, sekundäre und tertiäre Amine. Ammoniak ist eine erfindungsgemäß ganz besonders bevorzugte Base.

Erfindungsgemäß bevorzugte Puffersysteme sind beispielsweise das Tris-HCl-Puffersystem (Tris(hydroxymethyl)-aminomethan/Hydrogenchlorid) sowie Kalium- oder Natrium-Phophsat-Puffer (Mischung von K₂HPO₄ mit KH₂PO₄ beziehungsweise Na₂HPO₄ mit NaH₂PO₄, im pH-Bereich 7-7,8).

Neben den pH-Regulatoren können die erfindungsgemäßen Pflanzensäfte auch geeignete Stabilisatoren enthalten. Als ein geeigneter Stabilisator sei beispielsweise Phenylmethylsulfonylfluorid (PMSF) oder der kommerziell erhältlich Protease-Inhibitor-Mix, der von der Firma Sigma vertrieben wird, genannt. PMSF wird erfindungsgemäß bevorzugt in einer Konzentration von 0,1 bis 10mM eingesetzt, eine Konzentration von 0,5 bis 1,5mM ist erfindungsgemäß ganz besonders bevorzugt.

Die Mittel gemäß zweitem Gegenstand der vorliegenden Erfindung, enthaltend die Enzymfraktion sowie die natürlichen Aromastoffe eines Pflanzensaftes, können auf dem gleichen Wege gewonnen werden, wie dies oben für die Pflanzensäfte beschrieben wurde. Allerdings ist im Rahmen dieses Gegenstandes eine anschließende Aufreinigung des Pflanzensaftes nicht ausgeschlossen. Dies kann erfindungsgemäß beispielsweise durch eine Gel-Chromatograpie erfolgen, wobei die Inhaltsstoffe aufgrund ihrer Größe voneinander getrennt werden. Weiterhin ist prinzipiell ein Einsatz aller gängigen Chromatographie-Methoden denkbar, wie beispielsweise z.B. die Anionen- oder Kationen-Austausch-Chromatographie, bei der die Komponenten aufgrund ihrer Nettoladung voneinander getrennt werden.

Im Rahmen dieses Gegenstandes der vorliegenden Erfindung ist auch eine Gewinnung des Pflanzensaftes durch wässrige Extraktion denkbar, die üblicherweise so durchgeführt wird, dass die mechanisch zerkleinerten Pflanzenteile mit Wasser (bis höchstens 40°C) versetzt und durchgerührt werden, so dass sich durch die Erhöhung des Volumens der wässrigen Phase mehr Stoffe aus dem Pflanzengewebe im Wasser lösen.

Bevorzugte Pflanzenfrüchte im Sinne der vorliegenden Erfindung sind Früchte, die einen Wasseranteil von mehr als 50Gew.-% aufweisen. Erfindungsgemäß sind insbesondere die essbaren Früchte besonders bevorzugt.

Im Rahmen einer ersten Ausgestaltungsform der vorliegenden Erfindung werden Säfte eingesetzt, die aus Zitrusfrüchten gewonnen werden. Erfindungsgemäß haben sich beispielsweise die Zitrusfrüchte Zitrone, Orange, Mandarine, Pampelmuse, Grapefruit, Pomeranze und Limette als geeignet erwiesen. Zitrone, Orange und Grapefruit sind erfindungsgemäß besonders geeignet zur Gewinnung von Pflanzensäften.

Im Rahmen einer zweiten Ausgestaltungsform der vorliegenden Erfindung werden Säfte eingesetzt, die aus den Früchten von Nachtschattengewächsen gewonnen werden.

Erfindungsgemäß bevorzugte Nachtschattengewächse sind Kartoffel, Tomate, Stechapfel und Tollkirsche.

Im Rahmen einer dritten Ausgestaltungsform der vorliegenden Erfindung werden Säfte eingesetzt, die aus Früchten von Rosengewächsen gewonnen werden. Erfindungsgemäß bevorzugte Beispiele sind Pfirsiche, Nektarinen, Äpfel, Birnen, Pflaumen, Himbeeren, Brombeeren, Weißdorn, Hagebutten, Aprikosen, Mirabellen, Quitten sowie Erdbeeren.

Der Pflanzensaft kann erfindungsgemäß mit weiteren kosmetisch akzeptablen Komponenten versetzt werden.

In einer ersten Ausführungsform dieses Gegenstandes der vorliegenden Erfindung kann der anwendungsbereite Pflanzensaft daher Konservierungsmittel zur Hemmung von Mikroorganismen enthalten. Als erfindungsgemäß bevorzugte Konservierungsmittel haben sich beispielsweise Natriumazid, Parabene und Ethanol erwiesen. Besonders bevorzugte Konservierungsmittel sind erfindungsgemäß Natriumazid sowie die Parabene.

In einer zweiten Ausführungsform dieses Gegenstandes der vorliegenden Erfindung kann der Pflanzensaft kosmetisch verträgliche Enzymstabilisatoren enthalten. Als erfindungsgemäß bevorzugte Enzymstabilisatoren seien an dieser Stelle Polyethylenglykol und Glycerin genannt. Die erfindungsgemäßen Mittel enthalten die Enzymstabilisatoren bevorzugt in Mengen von 10 bis 50 Gew.-%, Konzentrationen von 15 bis 25% sind erfindungsgemäß besonders bevorzugt.

In einer dritten Ausführungsform dieses Gegenstandes der vorliegenden Erfindung kann dem Mittel, enthaltend den Pflanzensaft, ein Wirkstoff zugesetzt werden, der die natürlich vorkommenden Antioxidantien des Pflanzensaftes zerstören kann. Im Rahmen einer ganz besonders bevorzugten Ausführungsform der vorliegenden Erfindung, wird dieser Wirkstoff erst unmittelbar vor der Anwendung auf dem Haar zu den übrigen Bestandteilen der Anwendungszubereitung hinzugefügt.

Ein bevorzugter Wirkstoff im Sinne der vorliegenden Erfindung ist Wasserstoffperoxid. Weiterhin kommen Persulfate, Chlorite oder Anlagerungsprodukte von Wasserstoffperoxid an Harnstoff, Melamin sowie Natriumborat in Frage. Wasserstoffperoxid wird in den erfindungsgemäßen Mitteln vorzugsweise in einer Konzentration von 0,1 bis 2 Gew.-% eingesetzt.

Weiterhin können die erfindungsgemäßen Mittel einen Anfärbefarbstoff enthalten, damit das Produkt auch nach längerer Lagerung noch eine definierte und attraktive Farbe aufweist.

Hinsichtlich weiterer Komponenten und der Lagerung der Pflanzensäfte sei an dieser Stelle auf die in der Kosmetik üblichen Prozesse verwiesen.

Hinsichtlich der in den erfindungsgemäßen Färbemitteln eingesetzten Farbstoffvorprodukte unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

Als Farbstoffvorprodukt enthalten die erfindungsgemäßen Färbemittel bevorzugter Weise mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 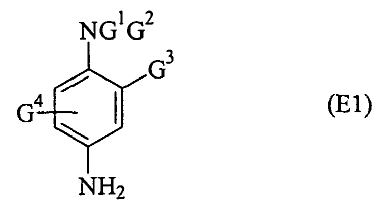 wobei
- G¹ steht für ein Wasserstoffatom, einen C₁- bis C₄-Alkylrest, einen C₁- bis C₄₋Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄₋Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄₋Acetylaminoalkoxyrest, einen C₁- bis C₄- Mesylaminoalkoxyrest oder einen C₁- bis C₄₋Carbamoylaminoalkoxyrest;
- G⁴ steht für ein Wasserstoffatom, ein Halogenatom oder einen C₁- bis C₄₋Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄₋Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄₋Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 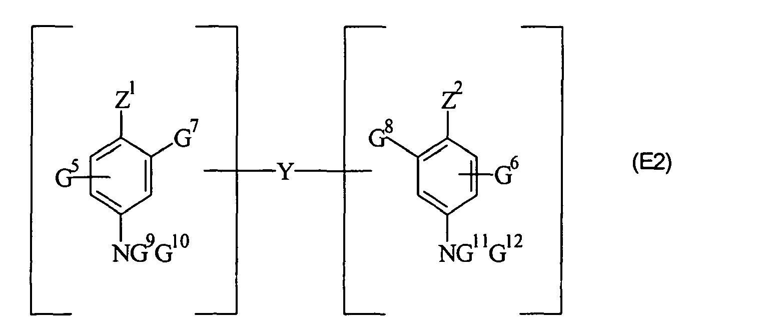
wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈₋Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄₋Alkylrest,
   mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4-aminophenyl)-1,3-diamino-propan-2-ol, N,N'-Bis-(β-hydroxyethyl)-N,N'-bis-(4-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4-amino-3-methylphenyl)-ethylendiamin, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, N,N'-Bis-(2-hydroxy-5-aminobenzyl)-piperazin, N-(4-Aminophenyl)-p-phenylendiamin und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 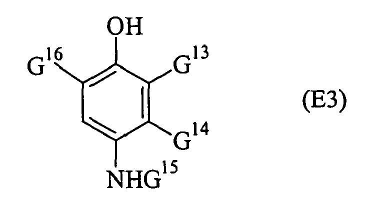
wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁₋bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄₋Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄₋Cyanoalkylrest,
- G¹⁵ steht für Wasserstoff, einen C₁- bis C₄-Alkylrest, einen C₁- bis C₄₋Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropyl pyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 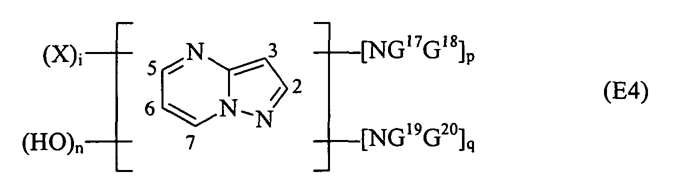
wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂₋bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁₋bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁₋bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄₋Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄₋hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

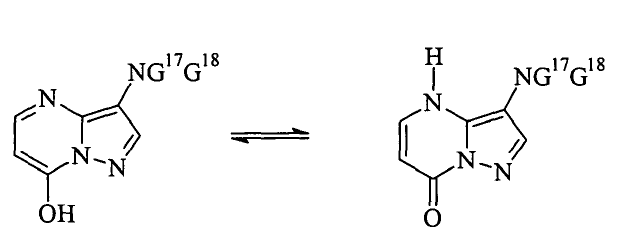

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

In einer weiteren Ausführungsform der vorliegenden Erfindung können die Färbemittel als Farbstoffvorprodukt mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (la), 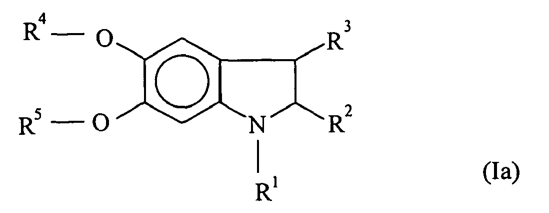 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 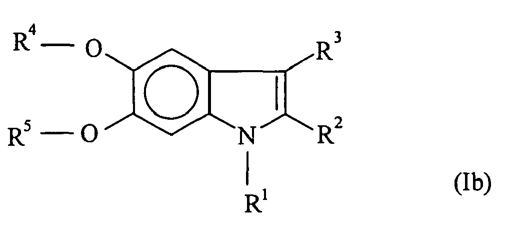 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Die erfindungsgemäßen Färbemittel können in einer weiteren bevorzugten Ausführungsform der vorliegenden Erfindung neben den Farbstoffvorprodukten zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quatemäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

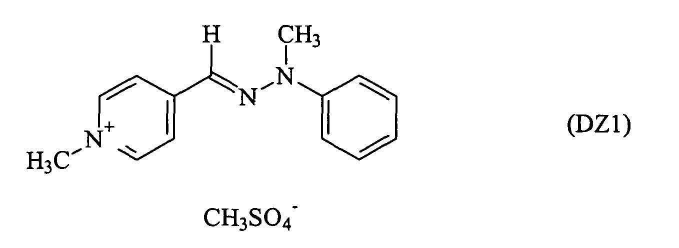

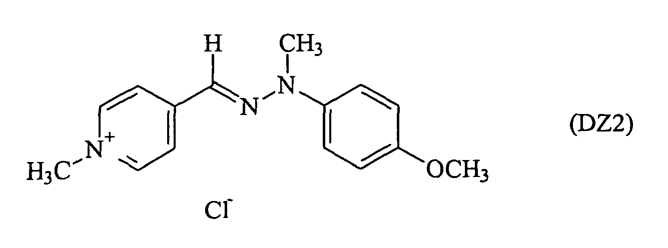

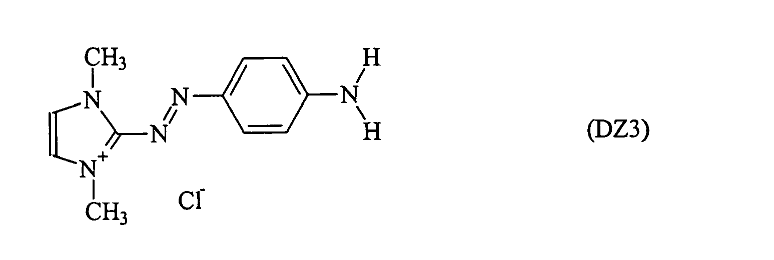

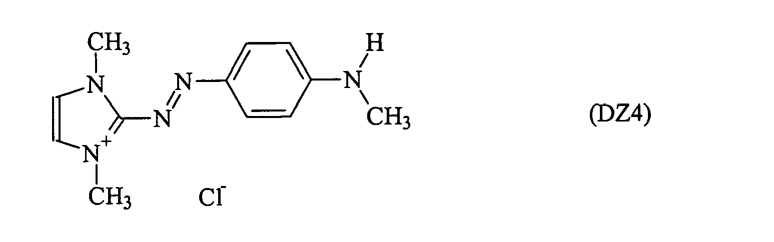

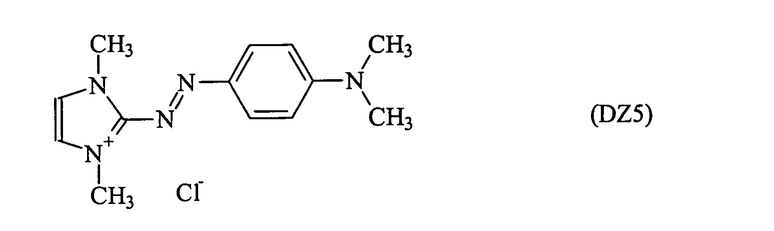

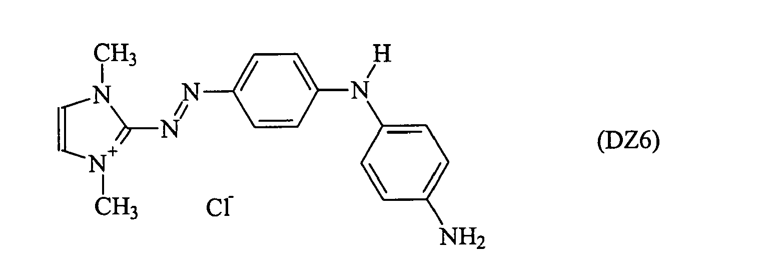

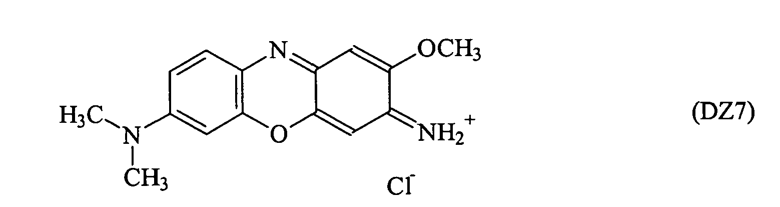

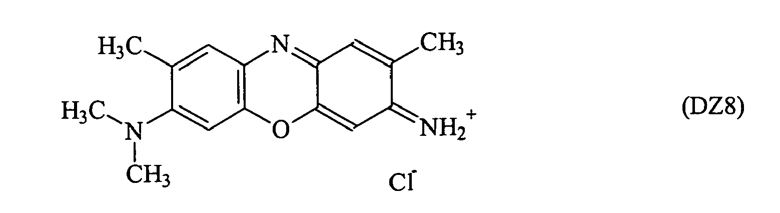

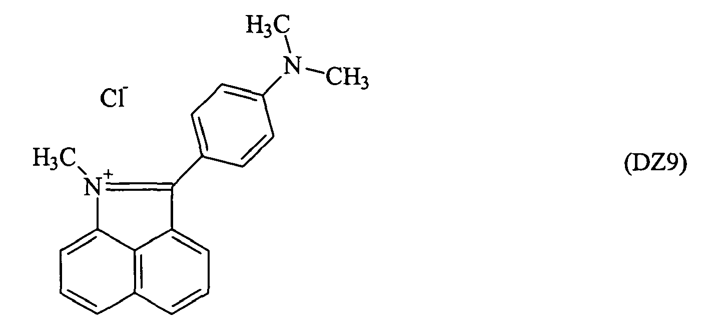

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Farbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Der Fachmann kann einen eventuellen Einfluss der verschiedenen Tenside auf die Aktivität des erfindungsgemäßen Pflanzensaftes gegebenenfalls durch einfache Vorversuche überprüfen.

In einer bevorzugten Ausführungsform der vorliegenden Erfindung wird in den Mitteln zur Färbung keratinischer Fasern eine Kombination aus anionischen und nichtionischen Tensiden oder eine Kombination aus anionischen und amphoteren Tensiden eingesetzt.

Es hat sich aber in Einzelfällen als vorteilhaft erwiesen, die Tenside aus amphoteren oder nichtionischen Tensiden auszuwählen, da diese in der Regel den erfindungsgemäßen Färbeprozess weniger beeinflussen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quatemäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol, Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Das eigentliche Haarfärbemittel wird zweckmäßigerweise unmittelbar vor der Anwendung durch Mischung der Zubereitung des Pflanzensaftes mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die eigentliche oxidative Färbung der Fasern kann grundsätzlich mit dem Pflanzensaft als alleinigem Oxidationsmittel erfolgen. In seltenen Fällen wird jedoch ein weiteres, chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Ein dritter Gegenstand der vorliegenden Anmeldung ist ein 2-Komponentenmittel zum Färben keratinischer Fasern, das aus einer ersten Komponente (A), enthaltend mindestens ein Farbstoffvorprodukt, und einer zweiten Komponente (B), enthaltend mindestens einen Pflanzensaft, besteht. Bezüglich der Definitionen der einzelnen Komponenten sei an dieser Stelle auf die obigen Ausführungen verwiesen.

Ein vierter Gegenstand der vorliegenden Anmeldung ist ein 3-Komponentenmittel zur Färbung keratinischer Fasern, das aus einer erste Komponente (A), enthaltend mindestens ein Farbstoffvorprodukt, einer zweite Komponente (B), enthaltend mindestens einen Pflanzensaft, und einer dritte Komponente (C), enthaltend mindestens einen Wirkstoff, der die Antioxidantien des Pflanzensaftes zerstören kann, besteht.

Im Rahmen des dritten und vierten Gegenstandes der vorliegenden Anmeldung kann es bevorzugt sein, wenn die zweite Komponente (B) des Mehrkomponentenmittel weiterhin mindestens einen Stabilisator enthält. Hinsichtlich der Details der erfindungsgemäß geeigneten Stabilisatoren sei auch an dieser Stelle auf die obigen Ausführungen verwiesen.

Ein fünfter Gegenstand der vorliegenden Erfindung ist ein Verfahren zur Färbung keratinischer Fasern, bei dem eines der beschriebenen erfindungsgemäßen Mittel auf die Fasern aufgetragen wird und nach einer Einwirkzeit wieder abgespült wird.

Ein sechster Gegenstand der vorliegenden Erfindung ist die Verwendung eines Pflanzensaftes zur Farbentwicklung aus an sich ungefärbten Farbstoffvorprodukten im Rahmen einer oxidativen Färbung keratinischer Fasern.

### Ausführungsbeispiele

### 1 Beispiel 1:

### 1.1 Herstellung von Pfirsichsaft:

270 g Pfirsich wurden ungeschält in einem Haushaltsmixer zerkleinert (mehrmals kurz auf höchster Stufe gemixt). Nach Zentrifugation (400 upm für 15 Minuten) wurde der noch leicht trübe Überstand abgenommen und filtriert. Man erhielt einen klaren Saft, der in einem Volumenverhältnis von 1:1 mit einem Tris-HCl Puffer (pH7.5, 0.1 M) verdünnt wurde. Das Pellet und der Filterkuchen wurden verworfen. Der Saft wurde bei 4°C im Kühlschrank gelagert.

### 1.2 Herstellung der Cremegrundlage

Aus den folgenden Komponenten wurde die Cremegrundlage erstellt:

| | |
|---|---|
| Hydrenol® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

| | |
|---|---|
| ¹ C₁₆₋₁₈-Fettalkohol (INCI-Bezeichnung: Cetearyl alcohol) (Cognis) | |
| ² C₁₂₋₁₈-Fettalkohol (INCI-Bezeichnung: Coconut alcohol) (Cognis) | |
| ³ Cetylstearylalkohol mit ca. 20 EO-Einheiten (INCI-Bezeichnung: Ceteareth-20) (Cognis) | |
| ⁴ Laurylethersulfat, Natriumsalz (ca. 27,5% Aktivsubstanz; INCI-Bezeichnung: Sodium Laureth Sulfate) (Cognis) | |
| ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) | |

Die Substanzen Hydrenol® D, Lorol® und Eumulgin® B2 wurden bei 80°C aufgeschmolzen, mit dem 80°C heißem Wasser, enthaltend Texapon® NSO und Dehyton® K, vermischt und unter starkem Rühren emulgiert. Danach wurde die Emulsion unter schwachem Rühren abgekühlt.

### 1.3 Herstellung der Anwendungszubereitungen

Es wurden die folgenden Anwendungszubereitungen hergestellt

| Komponenten | Versuch A | Versuch B | Versuch C | Versuch D |
|---|---|---|---|---|
| p-Toluylendiamin-sulfat | 0,00125 mol | 0,00125 mol | 0,00125 mol | 0,00125 mol |
| Resorcin | 0,00125 mol | 0,00125 mol | 0,00125 mol | 0,00125 mol |
| Färbecreme (siehe Pkt. 1.2) | 50 g | 50 g | 50 g | 50 g |
| Pfirsichsaft (siehe Pkt. 1.1) | 25ml | 25ml | -- | -- |
| H₂O₂ (30%ige wässrige Lösung) | -- | 0,08ml | 2,5ml | -- |
| Tris-HCl-Puffer (0.1 M, pH 7.5) | ad 100 g | ad 100 g | ad 100 g | ad 100 g |

Die erfindungsgemäße Anwendungszubereitung A enthält als Oxidationsmittel Pfirsichsaft. Die ebenfalls erfindungsgemäße Anwendungszubereitung B enthält neben dem Pfirsichsaft geringe Mengen Wasserstoffperoxid zur Zerstörung der im Pfirsichsaft enthaltenen Antioxidantien im Pfirsichsaft. Die Anwendungszubereitung C dient als Positiv-Kontrolle und enthält das herkömmliche Oxidationsmittel Wasserstoffperoxid. Färbezubereitung D diente als Negativ-Kontrolle ohne Zusatz eines Oxidationsmittels.

### 1.4 Ausfärbung

Jeweils 16g der Anwendungszubereitungen A bis D wurden auf je eine 0,5g schweren und 6cm langen Haarsträhne aus menschlichem Kerling-Haar (80% Grau-Anteil) aufgetragen. Nach einer Einwirkzeit von 30 Minuten bei Raumtemperatur wurden die Strähnen mit lauwarmem Wasser gespült und dann an der Luft getrocknet.

### 1.5 Ergebnis:

Die Bewertung der Farbtöne erfolgte im Tageslicht nach der Farbskala im Farbkatalog (Taschenlexikon der Farben, A. Kornerup u. J.H. Wanscher, Muster-Schmidt-Verlag, 3. unveränderte Auflage 1981). In der folgenden Tabelle I sind die erreichten Farbintensitäten für die einzelnen Anwendungszubereitungen zusammengestellt:

**Tabelle I:**

| Färbezubereitung | Farbintensität des Haares nach Farbkatalog |
|---|---|
| A (erfindungsgemäß) | 5F8 umbra (roh) |
| B (erfindungsgemäß) | 6F7 kastanienbraun |
| C (Vergleichsbeispiel) | 5F6 tabakbraun |
| D (Vergleichsbeispiel) | 4C5 blond |

Mit der erfindungsgemäßen Anwendungszubereitung A wurde eine intensive Ausfärbung erhalten. Die Intensität der Ausfärbung konnte mit der erfindungsgemäßen Anwendungszubereitung B noch gesteigert werden. Die Ausfärbung mit der erfindungsgemäßen Anwendungszubereitung B ist in ihrer Intensität mit der Wirkung von Wasserstoffperoxid vergleichbar (Anwendungsrezeptur C). Die Negativ-Kontrolle D (ohne Zusatz eines Oxidationsmittels) ergab keine nennenswerte Ausfärbung.

### 2 Beispiel 2

### 2.1 Herstellung des Tomatensafts

185 g Tomaten wurden im Haushaltsmixer zerkleinert (mehrmals kurz auf höchster Stufe gemixt) und mit 70 mg PMSF (gelöst in Isopropanol) versetzt. Es wurden 3ml einer 1 molaren NaOH-Lösung zugegeben. Anschließend wurde 5 Minuten lang bei Raumtemperatur gerührt. Durch Zentrifugation (4000 upm für 15 Minuten) wurden die Zellreste vom wässrigen Überstand abgetrennt. Nach der Zentrifugation wurden drei Phasen erhalten, eine untere Phase, die Feststoffe enthielt, eine mittlere flüssige Phase und eine obere Phase, die ebenfalls Feststoff enthielt. Die mittlere klare, leicht gelbliche Phase bildete den Tomatensaft. Er wurde vorsichtig abpipettiert und bei 4°C im Kühlschrank aufbewahrt.

### 2.2 Herstellung der Anwendungszubereitungen

Es wurden die folgenden Anwendungszubereitungen hergestellt:

| Komponenten | Versuch E | Versuch F | Versuch G | Versuch H |
|---|---|---|---|---|
| p-Aminophenol | 0,00125 mol | 0,00125 mol | 0,00125 mol | 0,00125 mol |
| 5-Amino-2-methylphenol | 0,00125 mol | 0,00125 mol | 0,00125 mol | 0,00125 mol |
| Färbecreme (siehe Pkt. 1.2) | 50 g | 50 g | 50 g | 50 g |
| Tomatensaft (siehe PKT. 2.1) | 25 ml | --- | --- | --- |
| H₂O₂ (30%ig in Wasser) | 25 µl | 25 µl | 1 ml | --- |
| NH₄OH (1,5 Gew.-%ig) | ad pH 8,3 | ad pH 8,3 | Ad pH 8,3 | ad pH 8,3 |
| Wasser | ad 100g | ad 100g | ad 100g | ad 100g |

25 ml des Tomatensafts wurden kurz vor der Anwendung 25 µl einer 30%igen wässrigen Wasserstoffperoxidlösung unter Rühren zugesetzt. Der so behandelte Tomatensaft wurde in die Anwendungszubereitung E eingearbeitet. Zum Nachweis der Wirkung dieser geringen Wasserstoffperoxidkonzentration als Oxidationsmittel im Rahmen der Ausfärbung enthielt die Anwendungszubereitung F die gleiche Menge Wasserstoffperoxid als alleiniges Oxidationsmittel. Als Kontrolle für die Leistung eines chemischen Oxidationsmittels wurde die Anwendungszubereitung G mit 1 % Wasserstoffperoxid formuliert. Als Kontrolle (Ausfärbung ohne Zusatz eines Oxidationsmittels) diente die Anwendungszubereitung H.

### 2.3 Ausfärbung

Der Färbevorgang wurde wie unter Pkt. 1.4 beschrieben durchgeführt.

### 2.4 Ergebnisse

Die Bewertung der Farbtöne wurde wie unter Pkt. 1.5 beschrieben vorgenommen. In der folgenden Tabelle II sind die erreichten Farbintensitäten für die einzelnen Anwendungszubereitungen zusammengefasst:

**Tabelle II:**

| Färbezubereitung | Farbintensität des Haares nach Farbkatalog |
|---|---|
| E (erfindungsgemäß) | 6A8 dunkel-orange/chrom-orange |
| F (Vergleichsbeispiel) | 5A4 hell-orange |
| G (Vergleichsbeispiel) | 6A7 persisch-orange |
| H (Vergleichsbeispiel) | 4A4 hellgelb |

Die mit der erfindungsgemäßen Anwendungszubereitung E erzielte Färbung ist wesentlich intensiver als die mit der Anwendungszubereitung F erzielte Färbung. In der Intensität ist sie mindestens vergleichbar mit der Wirkung von Wasserstoffperoxid (Anwendungszubereitung G). Sowohl die Anwendungszubereitung F (geringe Konzentration eines Oxidationsmittels) als auch die Anwendungszubereitung H (ohne Zusatz eines Oxidationsmittels) ergeben nur sehr geringe Farbintensitäten.

### 3 Beispiel 3

### 3.1 Herstellung von Bananensaft

Das Fruchtfleisch von 2 Bananen wurde im Haushaltsmixer zerkleinert (mehrmals kurz auf höchster Stufe gemixt) und mit 70 mg PMSF (Inhibitor von Proteasen, gelöst in Isopropanol) versetzt und zur leichteren Weiterverarbeitung in einem Volumenverhältnis von 1:1 mit einem Tris-HCl Puffer (pH 7.5, 0.1 M) verdünnt. Nach Zentrifugation (4000 upm für 15 Minuten) wurde der noch leicht trübe Überstand abgenommen und filtriert. Es wurde ein klarer Saft erhalten. Das Pellet sowie die Schalen wurden verworfen. Der Saft wurde bei 4°C im Kühlschrank gelagert.

### 3.2 Herstellung der Anwendungszubereitungen

Es wurden die folgenden Anwendungszubereitungen hergestellt

| Komponenten | Versuch I | Versuch J | Versuch K |
|---|---|---|---|
| p-Toluylendiamin-sulfat | 0,00125 mol | 0,00125 mol | 0,00125 mol |
| Resorcin | 0,00125 mol | 0,00125 mol | 0,00125 mol |
| Färbecreme (siehe Pkt. 1.2) | 50 g | 50 g | 50 g |
| Bananensaft (siehe Pkt. 3.1) | 25 ml | --- | --- |
| H₂O₂ | 0,08 ml | 1 ml | --- |
| Tris-HCl-Puffer (0.1 M, pH 7.5) | ad 100 g | ad 100 g | ad 100 g |

Der Bananensaft wurde zum Abfangen von Antioxidantien mit Wasserstoffperoxid vorbehandelt (Anwendungszubereitung I). Anwendungszubereitung J diente als Positiv-Kontrolle. Sie enthielt 1 % H₂O₂. Anwendungszubereitung K diente als Negativ-Kontrolle. Sie enthielt kein Oxidationsmittel.

### 3.3 Ausfärbung

Der Färbevorgang wurde wie unter Pkt. 1.4 beschrieben durchgeführt.

### 3.4 Ergebnis

Die Bewertung der Farbtöne wurde wie unter Pkt. 1.5 beschrieben vorgenommen. In der folgenden Tabelle III sind die erreichten Farbintensitäten für die einzelnen Anwendungszubereitungen zusammengefasst:

**Tabelle III:**

| Färbezubereitung | Farbintensität des Haares nach Farbkatalog |
|---|---|
| I (erfindungsgemäß) | 6E4 graubraun |
| J (Vergleichsbeispiel) | 5F6 tabakbraun |
| K (Vergleichsbeispiel) | 4C5 blond |

Die mit der Anwendungszubereitung I erzielte Farbe ist in der Intensität mindestens vergleichbar mit der Wirkung von Wasserstoffperoxid (Anwendungszubereitung J). Mit der Anwendungszubereitung K (ohne Zusatz eines Oxidationsmittel) wird keine nennenswerte Farbe erzielt.

## Patentansprüche

1. Mittel zur Färbung keratinischer Fasern, enthaltend in einem physiologisch akzeptablen Medium mindestens ein Farbstoffvorprodukt sowie mindestens einen Pflanzensaft.

2. Mittel zur Färbung keratinischer Fasern, enthaltend in einem physiologisch akzeptablen Medium mindestens ein Farbstoffvorprodukt sowie zumindest die Enzymfraktion sowie die natürlichen Aromastoffe eines Pflanzensaftes.

3. Mittel nach einem der Ansprüche 1 oder 2, **dadurch gekennzeichnet, dass** der Pflanzensaft aus einer Zitrusfrucht gewonnen wird.

4. Mittel nach einem der Ansprüche 1 oder 2, **dadurch gekennzeichnet, dass** der Pflanzensaft aus einer Frucht eines Nachtschattengewächses gewonnen wird.

5. Mittel nach einem der Ansprüche 1 oder 2, **dadurch gekennzeichnet, dass** der Pflanzensaft aus einer Frucht eines Rosengewächses gewonnen wird.

6. Mittel nach einem der Ansprüche 1 bis 5, **dadurch gekennzeichnet, dass** der Pflanzensaft weiterhin mindestens ein Konservierungsmittel enthält.

7. Mittel nach einem der Ansprüche 1 bis 6, **dadurch gekennzeichnet, dass** der Pflanzensaft weiterhin mindestens einen kosmetische verträglichen Enzymstabilisator enthält.

8. Mittel nach einem der Ansprüche 1 bis 7, **dadurch gekennzeichnet, dass** das Farbstoffvorprodukt eine Entwicklerkomponente ist.

9. Mittel nach einem der Ansprüche 1 bis 8, **dadurch gekennzeichnet, dass** das Farbstoffvorprodukt eine Vorstufe eines naturanalogen Farbstoffs ist.

10. Mittel nach einem der Ansprüche 8 oder 9, **dadurch gekennzeichnet, dass** weiterhin mindestens eine Kupplerkomponente enthalten ist.

11. Mittel nach einem der Ansprüche 1 bis 10, **dadurch gekennzeichnet, dass** es einen Wirkstoff enthält, der die Antioxidantien des Pflanzensaftes zerstören kann.

12. Mittel nach Anspruch 11, **dadurch gekennzeichnet, dass** dieser Wirkstoff Wasserstoffperoxid ist.

13. 2-Komponentenmittel zum Färben keratinischer Fasern, **dadurch gekennzeichnet, dass** es aus einer ersten Komponente (A), enthaltend mindestens ein Farbstoffvorprodukt, und einer zweiten Komponente (B), enthaltend mindestens einen Pflanzensaft, besteht.

14. 3-Komponentenmittel zur Färbung keratinischer Fasern, **dadurch gekennzeichnet, dass** es aus einer erste Komponente (A), enthaltend mindestens ein Farbstoffvorprodukt, einer zweite Komponente (B), enthaltend mindestens einen Pflanzensaft, und einer dritte Komponente (C), enthaltend mindestens einen Wirkstoff, der die Antioxidantien des Pflanzensaftes zerstören kann, besteht.

15. Mehrkomponentenmittel nach einem der Ansprüche 13 oder 14, **dadurch gekennzeichnet, dass** die zweite Komponente (B) weiterhin mindestens einen kosmetisch verträglichen Enzymstabilisator enthält.

16. Verfahren zur Färbung keratinischer Fasern, bei dem ein Mittel nach einem der Ansprüche 1 bis 12 auf die Fasern aufgetragen wird und nach einer Einwirkzeit wieder abgespült wird.

17. Verwendung eines Pflanzensaftes zur Farbentwicklung aus an sich ungefärbten Farbstoffvorprodukten im Rahmen einer oxidativen Färbung keratinischer Fasern.
